# EUROPEAN PATENT SPECIFICATION

(11) **EP 4 062 826 B1**
(45) Date of publication and mention of the grant of the patent: **07.02.2024**
(21) Application number: 22162217.8
(22) Date of filing: 15.03.2022
(51) Int. Cl.: A61B 5/097, A61B 5/085, A61M 16/00

(54) **VALVE HOUSING**
VENTILGEHÄUSE
BOÎTIER DE SOUPAPE

(30) Priority: 22.03.2021 GB 202103960
(43) Date of publication of application: 28.09.2022
(73) Proprietor: MD Diagnostics Limited, Otham, Kent ME15 8RJ (GB)
(72) Inventor: Quirke, Daniel, Otham, Kent ME15 8RJ (GB); Macdonald, Ian, Swanley, Kent BR8 7QD (GB)
(74) Representative: Bridle, Andrew Barry

(56) References cited:
- US-A1- 2016 279 362
- US-A1- 2019 299 055

## Description

The present application relates to valve housings and, in particular to valve housings for use with apparatus to measure inspiratory and expiratory lung pressure.

Apparatus which measure inspiratory and expiratory lung pressure require a user to either blow hard into a mouthpiece connected to the apparatus or to suck hard on the mouthpiece in order to measure the lung pressure they are able to exert. The mouthpiece typically forms part of a valve housing, wherein the housing includes a check valve that only permits air flow in a single direction. For example, when measuring expiratory lung pressure, the valve permits air to enter the valve housing, but prevents it from escaping from the housing. Thus, the patient can inhale via the mouthpiece, as the valve opens during inhalation, but when the patient blows into the mouthpiece, the valve closes and the expiratory lung pressure can be measured. Similarly, when measuring inspiratory lung pressure, the valve permits air to exit the valve housing, but prevents it from entering into the housing. Thus, the patient can exhale via the mouthpiece, as the valve opens during exhalation, but when the patient sucks on the mouthpiece, the valve closes and the inspiratory lung pressure can be measured.

Known valve housings typically also include a filter medium to prevent contamination of the apparatus. Such a valve housing is known from US 2016/279362 A1.

While known valve housings function adequately, they tend to be difficult to assemble. The present invention seeks to address or ameliorate this problem.

The invention is defined in the appended claims.

According to a first aspect of the invention, there is provided a valve housing for use in an apparatus for measuring inspiratory and/or expiratory lung pressure, the valve housing comprising a first body portion which defines a mouthpiece; a second body portion coupled to the first body portion and which defines a port; a filter medium disposed within a cavity defined by the first and second body portions; a pair of spaced apart valve support bodies defined by one of the first and second body portions, wherein each valve support body defines an air flow conduit that includes a support frame disposed within the conduit, the support frame having a first side and a second side; and a pair of valves, wherein the valves are selectively secured to the respective first sides of the support frame or are secured to the respective second sides of the support frame, wherein when the valves are secured to the first sides of the support frame, they open to permit air to exit the valve housing via the air flow conduits, but close to prevent air being drawn into the valve housing from the external environment; and when the valves are secured to the second sides of the support frame, they open to permit air to enter into the valve housing via the air flow conduits from an external environment, but close to prevent air within the housing from exiting the housing.

Thus, when the valves are secured to the respective first sides of the support frame, the valve housing may be used to measure inspiratory lung pressure; and when the valves are secured to the respective second sides of the support frame, the valve housing may be used to measure expiratory lung pressure.

The valve housing according to the present invention incorporates two valves that can be easily secured to the support frames, as the valve support bodies may be formed during the moulding or casting of the first or second body portions. This allows the relevant component to be manufactured using a two part tool which is significantly easier and more cost effective in terms of the production and assembly of the product.

The first and second body portions define between them a cavity which is in fluid communication with the mouthpiece, the port and each of the air flow conduits defined by the valve support bodies. Thus, the mouthpiece, the port and the air flow conduits defined by the valve support bodies are all open at both ends. Other than the mouthpiece, the port and each of the air flow conduits defined by the valve support bodies, the cavity defined by the first and second body portions is suitably airtight.

In an embodiment of the invention, the first and second body portions each have a generally circular cross-section. It tends to be easier to create an airtight seal between the first and second body portions when they both include circular lips.

The mouthpiece may be arranged co-axially with the first body portion. Additionally, the port may be arranged co-axially with the second body portion. Thus, the mouthpiece and the port may be co-axial. In this way, a flow path from the mouthpiece to the port is substantially linear.

In an embodiment of the invention, the valve support bodies are arranged radially outwards from the mouthpiece or the port. The permits easier access to the support frame located within the valve support bodies for the installation of the valves.

Suitably, the second body portion is coupled to the first body portion via an air-tight coupling. This helps to ensure that accurate measurements of inspiratory and expiratory lung pressure can be made. For example, the second body portion may be adhered to the first body portion, may be welded to the first body portion, may be moulded to the first body portion or any combination thereof in order to provide an airtight seal between the first and second body portions.

In order to provide a good seal between the first and second body portions, one of the body portions may include a substantially cylindrical axially projecting skirt which is arranged to overlie a cylindrical connecting portion of the other body portion, such that a seal is formed between the skirt of one of the body portions and the cylindrical connecting portion of the other body portion. Thus, an outwardly facing circumferential surface of the cylindrical connecting portion may sealingly engage an inwardly facing surface of the skirt.

Optionally, the filter medium is secured within the joint formed between the first and second body portions. In other words, one side of the filter medium may be engaged with one of the first and second body portions and the opposite side of the filter medium may be engaged with the other of the first and second body portions.

In a further embodiment of the invention, the cavity defined by the first and second body portions is in the form of a chamber having a longitudinal axis (i.e. an axis defined by the flow path between the mouthpiece and the port) and the filter medium extends perpendicular to the longitudinal axis of the chamber and covers the entirety of the cross-sectional area of the chamber (i.e., the sectional area of the chamber where the plane of the cross-section is orthogonal to the longitudinal axis). Thus, the filter medium suitably covers entirely the flow path between the mouthpiece and the port.

In this way, the filter medium will trap any contaminant particles entrained within the air flow between the mouthpiece and the port.

Suitably, the valves are check valves. In other words, the valves are one way valves that only permit air flow in a single direction. In the present invention, the direction of the air flow permitted by the valves may be determined by the side of the support frame on which the valves are located.

For example, the valves may be diaphragm check valves. In such valves, the periphery of the diaphragm may seal against a sealing surface when the air flow is in a first direction (i.e., a closed configuration), and may be displaced away from the sealing surface when the air flow is in the opposite direction (i.e., an open configuration) The diaphragm may be secured on one side (i.e. a "flap" valve) or it may be secured along a midpoint of the diaphragm (i.e., a "butterfly" valve).

In an embodiment of the invention, the diaphragm check valves each have a circular cross-section; the valve support bodies each have a circular cross-section; and each support frame includes a diametric strut to which is secured a respective one of the diaphragm check valves. In such embodiments, the support frames may each define a circumferential sealing surface with which a peripheral edge portion of the respective diaphragm sealingly engages when closed.

Such check valves are reliable, robust and easy to install.

In a yet further embodiment of the invention, it is the first body portion that defines the valve support bodies. In such embodiments, the mouthpiece may include a tubular or cylindrical mouthpiece body or a frustoconical mouthpiece body, and the valve support bodies may be arranged on opposite sides of the mouthpiece body. Thus, the valve support bodies may be disposed radially outwards from the mouthpiece body.

According to a second aspect of the invention, there is provided an apparatus for measuring inspiratory lung pressure, the apparatus including a valve housing according to the first aspect of the invention as defined anywhere herein; a conduit which fluidly couples the port of the valve housing to a pressure measuring component of the apparatus, wherein the valves of the valve housing are secured to the respective first sides of the support frame.

According to a third aspect of the invention, there is provided an apparatus for measuring expiratory lung pressure, the apparatus including a valve housing according to the first aspect of the invention as defined anywhere herein; a conduit which fluidly couples the port of the valve housing to a pressure measuring component of the apparatus, wherein the valves of the valve housing are secured to the respective second sides of the support frame.

The skilled person will appreciate that the pressure measuring component of the apparatus may be common to both the second and third aspects of the invention. In such a case, it is only the side of the support frame of the valve housing to which the valves are secured that is different.

Accordingly, a fourth aspect of the invention provides an apparatus for measuring inspiratory lung pressure and for measuring expiratory lung pressure, the apparatus including a first valve housing according to the first aspect of the invention as defined anywhere herein; a second valve housing according to the first aspect of the invention as defined anywhere herein; a conduit which fluidly couples the port of the first or second valve housing to a pressure measuring component of the apparatus, wherein the valves of the first valve housing are secured to the respective first sides of the support frame and the valves of the second valve housing are secured to the respective second sides of the support frame.

Thus, the fourth aspect of the invention may comprise a kit including a pressure measuring component, a conduit, a first valve housing and a second valve housing, wherein the appropriate valve housing is selected for use with the measuring component depending on the desired lung pressure to be measured.

The measuring components of the second, third and fourth aspects of the invention are well known components.

According to a fifth aspect of the invention, there is provided a method of measuring inspiratory lung pressure of a patient, the method comprising providing an apparatus as defined in the second or fourth aspects of the invention; the patient exhaling through the mouthpiece, wherein the valves open and permit the exhaled air to exit the valve housing; and the patient exerting an inspiratory action, wherein the valves close and the apparatus measures the inspiratory lung pressure.

According to a sixth aspect of the invention, there is provided a method of measuring expiratory lung pressure of a patient, the method comprising providing an apparatus as defined in the third or fourth aspects of the invention; the patient inhaling through the mouthpiece, wherein the valves open and permit air to enter the patient's lungs via the valve housing; and the patient exerting an expiratory action, wherein the valves close and the apparatus measures the expiratory lung pressure.

The skilled person will appreciate that the features described and defined in connection with the aspects of the invention and the embodiments thereof may be combined in any combination, regardless of whether the specific combination is expressly mentioned herein. Thus, all such combinations are considered to be made available to the skilled person.

An embodiment of the invention will now be described, by way of example only, with reference to the accompanying drawings in which:
Figure 1a shows a cross-sectional view through a valve housing according to a first embodiment of the first aspect of the invention;
Figure 1b shows an end view of the valve housing shown in Figure 1a from the mouthpiece end of the housing;
Figure 2a shows a cross-sectional view through a valve housing according to a second embodiment of the first aspect of the invention;
Figure 2b shows an end view of the valve housing shown in Figure 2a from the mouthpiece end of the housing;
Figure 3 shows a side view of the valve housing shown in Figures 1a, 1b, 2a and 2b; and
Figure 4 shows a perspective view of the valve housing shown in Figure 3.

For the avoidance of doubt, the skilled person will appreciate that in this specification, the terms "up", "down", "front", "rear", "upper", "lower", "width", etc. refer to the orientation of the components as found in the example when installed for normal use as shown in the Figures.

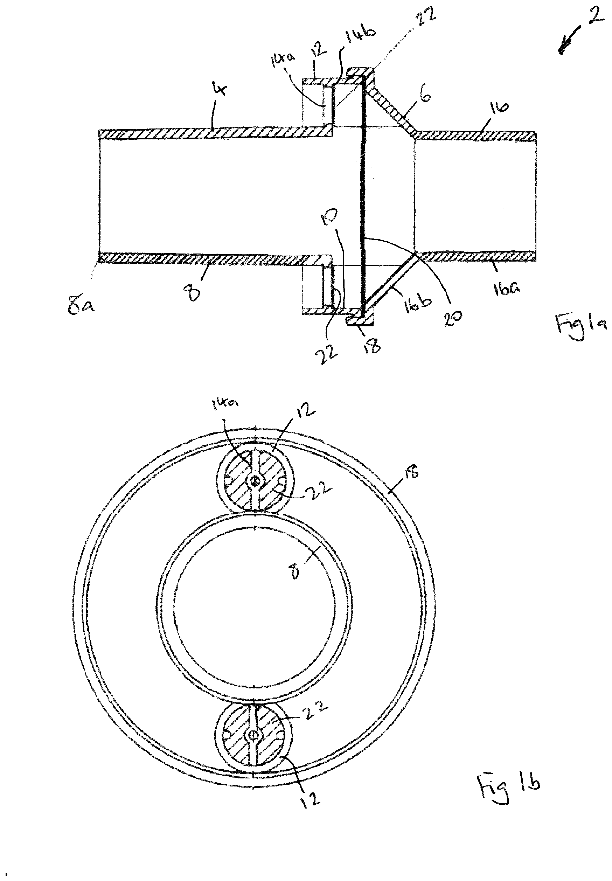

Figures 1a and 1b show a valve housing 2 according to a first embodiment of the invention. The valve housing 2 includes a first body portion 4 and a second body portion 6.

The first body portion 4 defines a cylindrical mouthpiece body 8 which is open at its distal end 8a and its proximal end opens into a cavity defined by the first and second body portions 4, 6.

The first body portion 4 further includes a cylindrical connecting portion 10 which projects away from the mouthpiece body 8.

A pair of opposed cylindrical valve support bodies 12 are defined on either side of the mouthpiece body 8. Each of the support bodies 12 include a support frame comprising a diametric support strut 14a and an annular sealing surface 14b.

The second body portion 6 includes a body 16, which includes a cylindrical port body portion 16a and an outwardly flared frustoconical portion 16b. The outwardly flared frustoconical portion 16b terminates in an axially extending skirt 18.

As shown in Figure 1a, the skirt 18 overlaps a portion of the cylindrical connecting portion 10, such that an inwardly facing surface of the skirt 18 engages an outwardly facing surface of the connecting portion 10. These engaged surfaces are welded together to form an air-tight seal between the first body portion 4 and the second body portion 6.

A filter medium 20 is secured between the first and second body portions 4, 6 and welded thereto to provide a contaminant barrier across the air flow channel defined by the first and second body portions 4, 6.

In this embodiment, a butterfly diaphragm valve 22 is secured to the diametric support strut 14a on a second side of each of the support frames. In this arrangement, a peripheral edge portion of each valve 22 lies adjacent to the annular sealing surface 14b. With the valves 22 secured to the second side of the support frames, they open when the pressure within the cavity defined by the first and second body portions 4, 6 drops below the air pressure external to the housing 2 (i.e. when a patient inhales through the mouthpiece body 8). When the patient then exerts an expiratory lung pressure, the valves 22 close and are sealed against the sealing surface 14b. An expiratory lung pressure measuring apparatus (not shown), which is connected to the valve housing 2 via a conduit (not shown) which is connected to the port body portion 16a can then measure the expiratory lung pressure.

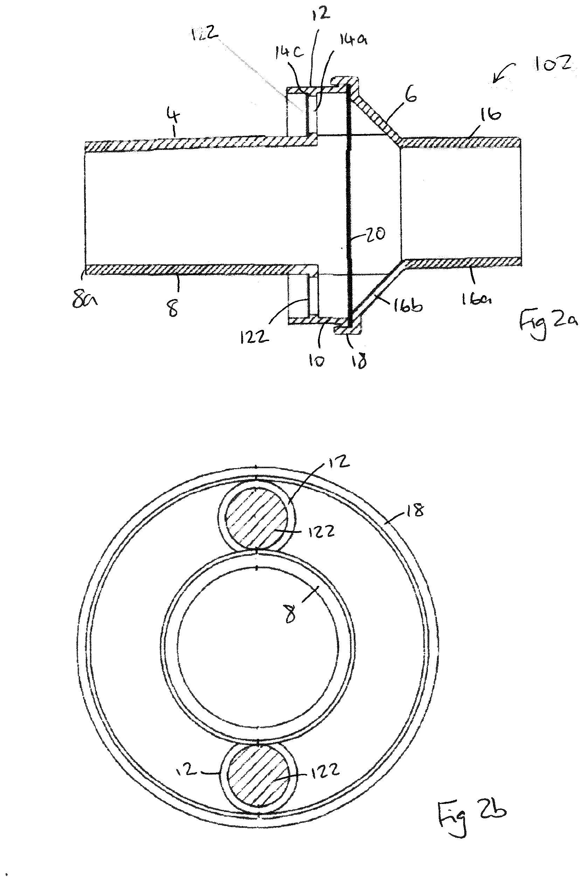

Figures 2a and 2b show a valve housing 102, which is identical to the valve housing 2, except that a pair of butterfly diaphragm valves 122 are secured to a first side of the support frame diametric support strut 14a. In this case, a peripheral edge portion of each of the valves 122 lies adjacent to an annular sealing surface 14c defined by the support frame. With the valves 122 secured to the first side of the support frames, they open when the pressure within the cavity defined by the first and second body portions 4, 6 increases above the air pressure external to the housing 2 (i.e. when a patient exhales through the mouthpiece body 8). When the patient then exerts an inspiratory lung pressure, the valves 122 close and are sealed against the sealing surface 14c. An inspiratory lung pressure measuring apparatus (not shown), which is connected to the valve housing 102 via a conduit (not shown) which is connected to the port body portion 16a can then measure the inspiratory lung pressure.

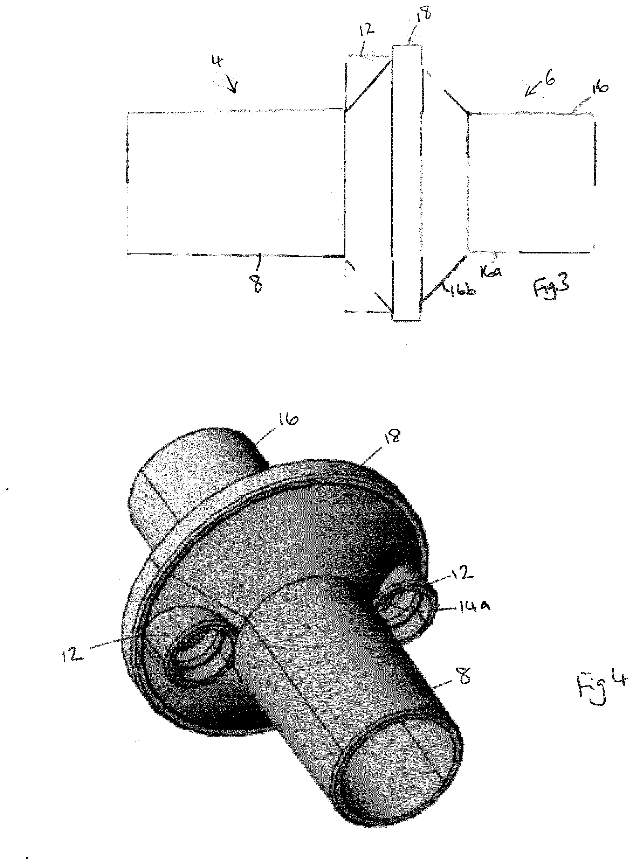

Figure 3 shows a side view of the valve housing 2, 102.

Figure 4 shows a perspective view of the valve housing 2, 102.

## Claims

1. A valve housing for use in an apparatus for measuring inspiratory and expiratory lung pressure, the valve housing comprising a first body portion which defines a mouthpiece; a second body portion coupled to the first body portion and which defines a port; a pair of spaced apart valve support bodies defined by one of the first and second body portions, wherein each valve support body defines an air flow conduit that includes a support frame disposed within the conduit, the support frame having a first side and a second side; and a pair of valves, wherein the valves are selectively secured to the respective first sides of the support frame or are secured to the respective second sides of the support frame, wherein when the valves are secured to the first sides of the support frame, they open to permit air to exit the valve housing via the air flow conduits, but close to prevent air being drawn into the valve housing from the external environment; and when the valves are secured to the second sides of the support frame, they open to permit air to enter into the valve housing via the air flow conduits from an external environment, but close to prevent air within the housing from exiting the housing,
**characterised in that** the valve housing further comprises a filter medium disposed between the first and second body portions.

2. A valve housing according to Claim 1, wherein the first and second body portions each have a generally circular cross-section.

3. A valve housing according to Claim 2, wherein the mouthpiece is arranged co-axially with the first body portion and the port is arranged co-axially with the second body portion.

4. A valve housing according to Claim 3, wherein the valve support bodies are arranged radially outwards from the mouthpiece or the port.

5. A valve housing according to any of Claims 1 to 4, wherein the second body portion is coupled to the first body portion via an air-tight coupling.

6. A valve housing according to Claim 5, wherein the second body portion is adhered to the first body portion, is welded to the first body portion, or is moulded to the first body portion.

7. A valve housing according to any of Claims 1 to 6, wherein the first and second body portions together define a chamber having a longitudinal axis and the filter medium extends perpendicular to the longitudinal axis of the chamber and covers the entirety of the cross-sectional area of the chamber.

8. A valve housing according to any of Claims 1 to 7, wherein the valves are check valves; optionally wherein the valves are diaphragm check valves.

9. A valve housing according to Claim 8, wherein the diaphragm check valves each have a circular cross-section; the valve support bodies each have a circular cross-section; and each support frame includes a diametric strut to which is secured a respective one of the diaphragm check valves.

10. A valve housing according to any of Claims 1 to 9, wherein the first body portion defines the valve support bodies.

11. A valve housing according to Claim 10, wherein the mouthpiece includes a tubular or frustoconical mouthpiece body and the valve support bodies are arranged on opposite sides of the mouthpiece body.

12. An apparatus for measuring inspiratory lung pressure, the apparatus including a valve housing according to any of Claims 1 to 11; a conduit which fluidly couples the port of the valve housing to a pressure measuring component of the apparatus, wherein the valves of the valve housing are secured to the respective first sides of the support frame.

13. An apparatus for measuring expiratory lung pressure, the apparatus including a valve housing according to any of Claims 1 to 11; a conduit which fluidly couples the port of the valve housing to a pressure measuring component of the apparatus, wherein the valves of the valve housing are secured to the respective second sides of the support frame.

14. A method of measuring inspiratory lung pressure of a patient, the method comprising providing an apparatus as defined in Claim 12; the patient exhaling through the mouthpiece, wherein the valves open and permit the exhaled air to exit the valve housing; and the patient exerting an inspiratory action, wherein the valves close and the apparatus measures the inspiratory lung pressure.

15. A method of measuring expiratory lung pressure of a patient, the method comprising providing an apparatus as defined in Claim 13; the patient inhaling through the mouthpiece, wherein the valves open and permit air to enter the patient's lungs via the valve housing; and the patient exerting an expiratory action, wherein the valves close and the apparatus measures the expiratory lung pressure.

## Patentansprüche

1. Ventilgehäuse zur Verwendung in einer Vorrichtung zur Messung eines Inspirations- und Exspirationsdrucks der Lungen, wobei das Ventilgehäuse einen ersten Körperabschnitt, der ein Mundstück definiert; einen zweiten Körperabschnitt, der an den ersten Körperabschnitt gekoppelt ist und der einen Anschluss definiert; ein Paar von beabstandeten Ventilstützkörpern, die durch den ersten und den zweiten Körperabschnitt definiert sind, wobei jeder Ventilstützkörper eine Luftstromleitung definiert, die einen Stützrahmen beinhaltet, der innerhalb der Leitung angeordnet ist, wobei der Stützrahmen eine erste Seite und eine zweite Seite aufweist; und ein Paar von Ventilen umfasst, wobei die Ventile selektiv an den jeweiligen ersten Seiten des Stützrahmens gesichert sind oder an den jeweiligen zweiten Seiten des Stützrahmens gesichert sind, wobei, wenn die Ventile an den ersten Seiten des Stützrahmens gesichert sind, sie sich öffnen, um zuzulassen, dass Luft mittels der Luftstromleitungen aus dem Ventilgehäuse austritt, sich jedoch schließen, um zu verhindern, dass Luft aus der Außenumgebung in das Ventilgehäuse gezogen wird; und wenn die Ventile an den zweiten Seiten des Stützrahmens gesichert sind, sie sich öffnen, um zuzulassen, dass Luft mittels der Luftstromleitungen in das Ventilgehäuse eintritt, sich jedoch schließen, um zu verhindern, dass Luft innerhalb des Gehäuses aus dem Gehäuse austritt, **dadurch gekennzeichnet, dass** das Ventilgehäuse weiterhin ein Filtermedium umfasst, das zwischen dem ersten und dem zweiten Körperabschnitt angeordnet ist.

2. Ventilgehäuse nach Anspruch 1, wobei der erste und der zweite Körperabschnitt jeweils einen allgemein kreisförmigen Querschnitt aufweisen.

3. Ventilgehäuse nach Anspruch 2, wobei das Mundstück koaxial mit dem ersten Körperabschnitt eingerichtet ist und der Anschluss koaxial mit dem zweiten Körperabschnitt eingerichtet ist.

4. Ventilgehäuse nach Anspruch 3, wobei die Ventilstützkörper von dem Mundstück oder dem Anschluss radial nach außen eingerichtet sind.

5. Ventilgehäuse nach einem der Ansprüche 1 bis 4, wobei der zweite Körperabschnitt mittels einer luftdichten Kopplung an den ersten Körperabschnitt gekoppelt ist.

6. Ventilgehäuse nach Anspruch 5, wobei der zweite Körperabschnitt an den ersten Körperabschnitt angeklebt ist, an den ersten Körperabschnitt geschweißt ist oder an den ersten Körperabschnitt angegossen ist.

7. Ventilgehäuse nach einem der Ansprüche 1 bis 6, wobei der erste und der zweite Körperabschnitt zusammen eine Kammer mit einer Längsachse definieren und das Filtermedium sich senkrecht zu der Längsachse der Kammer erstreckt und die Gesamtheit der Querschnittsfläche der Kammer abdeckt.

8. Ventilgehäuse nach einem der Ansprüche 1 bis 7, wobei die Ventile Rückflussventile sind; optional wobei die Ventile Membranrückflussventile sind.

9. Ventilgehäuse nach Anspruch 8, wobei die Membranrückflussventile jeweils einen kreisförmigen Querschnitt aufweisen; die Ventilstützkörper jeweils einen kreisförmigen Querschnitt aufweisen und jeder Stützrahmen eine diametrische Strebe beinhaltet, an der ein jeweiliges der Membranrückflussventile gesichert ist.

10. Ventilgehäuse nach einem der Ansprüche 1 bis 9, wobei der erste Körperabschnitt die Ventilstützkörper definiert.

11. Ventilgehäuse nach Anspruch 10, wobei das Mundstück einen röhrenförmigen oder kegelstumpfförmigen Mundstückkörper beinhaltet und die Ventilstützkörper auf entgegengesetzten Seiten des Mundstückkörpers eingerichtet sind.

12. Vorrichtung zur Messung eines Inspirationsdrucks der Lungen, wobei die Vorrichtung ein Ventilgehäuse nach einem der Ansprüche 1 bis 11; eine Leitung, die den Anschluss des Ventilgehäuses fluidmäßig an eine Druckmesskomponente der Vorrichtung koppelt, beinhaltet, wobei die Ventile des Ventilgehäuses an den jeweiligen ersten Seiten des Stützrahmens gesichert sind.

13. Vorrichtung zur Messung eines Exspirationsdrucks der Lungen, wobei die Vorrichtung ein Ventilgehäuse nach einem der Ansprüche 1 bis 11; eine Leitung, die den Anschluss des Ventilgehäuses fluidmäßig an eine Druckmesskomponente der Vorrichtung koppelt, beinhaltet, wobei die Ventile des Ventilgehäuses an den jeweiligen zweiten Seiten des Stützrahmens gesichert sind.

14. Verfahren zur Messung eines Inspirationsdrucks der Lungen eines Patienten, wobei das Verfahren ein Bereitstellen einer Vorrichtung nach Anspruch 12; ein Ausatmen des Patienten durch das Mundstück, wobei die Ventile sich öffnen und zulassen, dass die ausgeatmete Luft aus dem Ventilgehäuse austritt; und ein Ausüben einer Inspirationsaktion, wobei die Ventile sich schließen und die Vorrichtung den Inspirationsdruck der Lungen misst, umfasst.

15. Verfahren zur Messung eines Exspirationsdrucks der Lungen eines Patienten, wobei das Verfahren ein Bereitstellen einer Vorrichtung nach Anspruch 13; ein Einatmen des Patienten durch das Mundstück, wobei die Ventile sich öffnen und zulassen, dass Luft mittels des Ventilgehäuses in die Lungen des Patienten eintritt; und ein Ausüben einer Exspirationsaktion, wobei die Ventile sich schließen und die Vorrichtung den Exspirationsdruck der Lungen misst, umfasst.

## Revendications

1. Boîtier de soupape destiné à être utilisé dans un appareil de mesure de pression pulmonaire inspiratoire et expiratoire, le boîtier de soupape comprenant une première partie formant corps qui définit un embout buccal ; une seconde partie formant corps accouplée à la première partie formant corps et définissant un orifice ; une paire de corps de support de soupape espacés, définis par une des première et seconde parties formant corps, chaque corps de support de soupape définissant un conduit d'écoulement d'air qui comprend un cadre de support disposé à l'intérieur du conduit, le cadre de support ayant un premier côté et un second côté ; et une paire de soupapes, les soupapes étant fixées de manière sélective aux premiers côtés respectifs du cadre de support ou aux seconds côtés respectifs du cadre de support, les soupapes, lorsqu'elles sont fixées aux premiers côtés du cadre de support, s'ouvrant pour permettre à l'air de sortir du boîtier de soupape par les conduits d'écoulement d'air, mais se fermant pour empêcher l'air d'être aspiré dans le boîtier de soupape depuis l'environnement extérieur ; et les soupapes, lorsqu'elles sont fixées aux seconds côtés du cadre de support, s'ouvrant pour permettre à l'air de pénétrer dans le boîtier de soupape par les conduits d'écoulement d'air depuis un environnement extérieur, mais se fermant pour empêcher l'air à l'intérieur du boîtier de sortir du boîtier, le boîtier de soupape étant **caractérisé en ce qu'**il comprend en outre un milieu filtrant disposé entre les première et seconde parties formant corps.

2. Boîtier de soupape selon la revendication 1, dans lequel les première et seconde parties formant corps ont chacune une section transversale généralement circulaire.

3. Boîtier de soupape selon la revendication 2, dans lequel l'embout buccal est disposé coaxialement avec la première partie formant corps et l'orifice est disposé coaxialement avec la seconde partie formant corps.

4. Boîtier de soupape selon la revendication 3, dans lequel les corps de support de soupape sont disposés radialement vers l'extérieur de l'embout buccal ou de l'orifice.

5. Boîtier de soupape selon l'une quelconque des revendications 1 à 4, dans lequel la seconde partie formant corps est accouplée à la première partie formant corps par l'intermédiaire d'un accouplement étanche à l'air.

6. Boîtier de soupape selon la revendication 5, dans lequel la seconde partie formant corps est collée à la première partie formant corps, est soudée à la première partie formant corps ou est moulée à la première partie formant corps.

7. Boîtier de soupape selon l'une quelconque des revendications 1 à 6, dans lequel les première et seconde parties formant corps définissent ensemble une chambre ayant un axe longitudinal, et le milieu filtrant s'étend perpendiculairement à l'axe longitudinal de la chambre et couvre la totalité de la section transversale de la chambre.

8. Boîtier de soupape selon l'une quelconque des revendications 1 à 7, dans lequel les soupapes sont des clapets anti-retour ; éventuellement, dans lequel les soupapes sont des clapets anti-retour à membrane.

9. Boîtier de soupape selon la revendication 8, dans lequel les clapets anti-retour à membrane ont chacun une section transversale circulaire ; les corps de support de soupape ont chacun une section transversale circulaire ; et chaque cadre de support comprend une entretoise diamétrale à laquelle est fixé un clapet anti-retour à membrane respectif des clapets anti-retour à membrane.

10. Boîtier de soupape selon l'une quelconque des revendications 1 à 9, dans lequel la première partie formant corps définit les corps de support de soupape.

11. Boîtier de soupape selon la revendication 10, dans lequel l'embout buccal comprend un corps d'embout buccal tubulaire ou tronconique et les corps de support de soupape sont disposés sur des côtés opposés du corps d'embout buccal.

12. Appareil de mesure de pression pulmonaire inspiratoire, l'appareil comprenant un boîtier de soupape selon l'une quelconque des revendications 1 à 11 ; un conduit qui accouple fluidiquement l'orifice du boîtier de soupape à un composant de mesure de pression de l'appareil, les soupapes du boîtier de soupape étant fixées aux premiers côtés respectifs du cadre de support.

13. Appareil de mesure de pression pulmonaire expiratoire, l'appareil comprenant un boîtier de soupape selon l'une quelconque des revendications 1 à 11 ; un conduit qui accouple fluidiquement l'orifice du boîtier de soupape à un composant de mesure de pression de l'appareil, les soupapes du boîtier de soupape étant fixées aux seconds côtés respectifs du cadre de support.

14. Procédé de mesure de pression pulmonaire inspiratoire d'un patient, le procédé consistant à fournir un appareil selon la revendication 12 ; le patient expirant à travers l'embout buccal, les soupapes s'ouvrant et permettant à l'air expiré de sortir du boîtier de soupape ; et le patient exerçant une action inspiratoire, les soupapes se fermant et l'appareil mesurant la pression pulmonaire inspiratoire.

15. Procédé de mesure de pression pulmonaire expiratoire d'un patient, le procédé consistant à fournir un appareil selon la revendication 13 ; le patient inspirant à travers l'embout buccal, les soupapes s'ouvrant et permettant à l'air de pénétrer dans les poumons du patient par l'intermédiaire du boîtier de soupape ; et le patient exerçant une action expiratoire, les soupapes se fermant et l'appareil mesurant la pression pulmonaire expiratoire.
